# EUROPEAN PATENT SPECIFICATION

(11) **EP 2 340 837 B1**
(45) Date of publication and mention of the grant of the patent: **15.03.2017**
(21) Application number: 11155582.7
(22) Date of filing: 09.11.2006
(51) Int. Cl.: A61K 31/337, A61P 35/00

(54) **COMBINATION TREATMENT OF CANCER COMPRISING EGFR/HER2 INHIBITORS**
KOMBINATIONSBEHANDLUNG GEGEN KREBS MIT EGFR/HER2-HEMMERN
TRAITEMENT COMBINÉ DU CANCER COMPRENANT DES INHIBITEURS EGFR/HER2

(30) Priority: 11.11.2005 EP 05110669
(43) Date of publication of application: 06.07.2011
(62) Divisional of application: 06819380.4
(73) Proprietor: Boehringer Ingelheim International GmbH, 55216 Ingelheim am Rhein (DE); Boehringer Ingelheim Pharma GmbH & Co. KG, 55216 Ingelheim am Rhein (DE)
(72) Inventor: Solca, Flavio, 1230, Wien (AT); Amelsberg, Andree, Danbury, CT 06810 (US); Van Meel, Jacobus C.A., 2340, Moedling (AT); Baum, Anke, 1050, Wien (AT); Stehle, Gerd, 89584, Ehingen (DE)
(74) Representative: Simon, Elke Anna Maria

(56) References cited:
- WO-A-02/50043
- WO-A1-2006/018182
- WO-A2-2004/096224

## Description

The invention relates to a pharmaceutical composition suitable in therapy of cancer comprising effective amounts of:
(1) a compound **1** :
   4-[(3-chloro-4-fluorophenyl)amino]-6-{{[4-(N,N-dimethylamino)-1-oxo-2-buten-1-yl]amino}-7-((*S*)-tetrahydrofuran-3-yloxy)-quinazoline dimaleate; and
(2) at least a further chemotherapeutic agent **2** selected from the group consisting of docetaxel and paclitaxel;
optionally in combination with one or more pharmaceutically acceptable excipients, and optionally adapted for a co-treatment with radiotherapy or radio-immunotherapy, in the form of a combined preparation for simultaneous, separate or sequential use.

### Background of the invention

Compound **1** and related compounds are disclosed in WO 02/50043, WO 2004/074263 and WO 2005/037824 as dual inhibitors of erbb1 receptor (EGFR) and erbB2 (Her2/neu) receptor tyrosine kinases, suitable for the treatment of e.g. benign or malignant tumours, particularly tumours of epithelial and neuroepithelial origin, metastasisation and the abnormal proliferation of vascular endothelial cells (neoangiogenesis), for treating diseases of the airways and lungs which are accompanied by increased or altered production of mucus caused by stimulation by tyrosine kinases, as well as for treating diseases of the gastrointestinal tract and bile duct and gall bladder which are associated with disrupted activity of the tyrosine kinases. The disclosure of WO 02/50043, WO 2004/074263 and WO 2005/037824 includes preparation as well as pharmaceutical formulations of the compounds. Furthermore, it is known for treatment of tumour diseases that the compounds may be used in monotherapy or in conjunction with other anti-tumour therapeutic agents, for example in combination with topoisomerase inhibitors (e.g. etoposide), mitosis inhibitors (e.g. vinblastine), compounds which interact with nucleic acids (e.g. cis-platin, cyclophosphamide, adriamycin), hormone antagonists (e.g. tamoxifen), inhibitors of metabolic processes (e.g. 5-FU etc.), cytokines (e.g. interferons) or antibodies. Treatment of tumour diseases with the combination of the VEGFR inhibitor 3-Z-[1-(4-(N-((4-methyl-piperazin-1-yl)-methylcarbonyl)-N-methyl-amino)-anilino)-1-phenylmethylene]-6-methoxycarbonyl-2-indolinone and one of the dual EGFR/HER2 inhibitors 4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline (compound **1**) or 4-[(3-chloro-4-fluoro-phenyl)amino]-6-{[4-(homomorpholin-4-yl)-1-oxo-2-buten-1-yl]amino}-7-[(S)-(tetrahydrofuran-3-yl)oxy]-quinazoline, among many others, are disclosed in WO 2004/096224. WO 2006/018182 discloses combinations of Polo-like kinase (PLK) inhibitors with a broad range of anti-cancer drugs, inter alia such as small molecule VEGF receptor antagonists, growth factor (GF) receptor antagonists, inhibitors of the EGF receptor including compound **1** or small molecule inhibitors of the Ras/Raf/MAPK or PI3K/AKT pathways and others.

For the treatment of diseases of oncological nature, a large number of chemotherapeutic, immunotherapeutic or immunomodulatory, antiangiogenic or hormonal agents have already been suggested, which can be used as monotherapy (treatment with one agent) or as combination therapy (simultaneous, separate or sequential treatment with more than one agent) and/or which may be combined with radiotherapy or radio-immunotherapy. In this respect, chemotherapeutic agent means a naturally occurring, semi-synthetic or synthetic chemical compound which, alone or via further activation, for example with radiations in the case of radio-immunotherapy, inhibits or kills growing cells, and which can be used or is approved for use in the treatment of diseases of oncological nature, which are commonly also denominated as cancers. In the literature, these agents are generally classified according to their mechanism of action. In this matter, reference can be made, for example, to the classification made in "Cancer Chemotherapeutic Agents", American Chemical Society, 1995, W.O. Foye Ed.

The efficacy of chemotherapeutic agents can be improved by using combination therapies with other chemotherapeutic, immunotherapeutic, immunomodulatory, antiangiogenic or hormonal compounds. Combination therapies constitute the gold standard in many settings of cancer therapy.
Even if the concept of combining several therapeutic agents or therapies already has been suggested, and although various combination therapies are under investigation and in clinical trials, there is still a need for new and efficient therapeutic compositions for the treatment of cancer diseases, which show advantages over standard therapies.
It is the purpose of the present invention to provide a pharmaceutical composition suitable for combination therapy with the dual inhibitor compound **1** for the treatment of various cancer diseases.

### Summary of the Invention

It has been found that a combination therapy for treatment of various cancer diseases, especially of the specific cancer-subindications mentioned hereinafter, comprising coadministration to a patient and/or co-treatment of a patient with a pharmaceutical composition comprising effective amounts of:
(1) a compound **1**:
   4-[(3-chloro-4-fluorophenyl)amino]-6-{[4-(N,N-dimethylamino)-1-oxo-2-buten-1-yl]amino}-7-((*S*)-tetrahydrofuran-3-yloxy)-quinazoline dimaleate; and
(2) at least a further chemotherapeutic agent **2** selected from the group consisting of docetaxel and paclitaxel;
optionally in combination with one or more pharmaceutically acceptable excipients, and optionally adapted for a co-treatment with radiotherapy or radio-immunotherapy,
provides unexpected advantages, e.g. superior efficacy based on additive or synergistic effects and/or improved tolerability and reduced side effects of the treatment by the patient due, for example, to the administration of lower doses of the therapeutic agents involved reduced side effects.

The expression "patient" relates to a human or non-human mammalian patient suffering from cancer and thus in need of such treatment, preferably the patient is a human person. Furthermore, the expression "patient" should be understood to include such cancer patients carrying tumors with wild-type EGF receptor as well as pre-selected cancer patients with tumors harboring activating EGFR mutations. These can be located in the tyrosine kinase domain of the EGF receptor such as for instance the L858R or L861 point mutations in the activation loop (exon 21), or in-frame deletion/insertion mutations in the ELREA sequence (exon 19), or substitutions in G719 situated in the nucleotide binding loop (exon 18). Additional activating mutations have been reported in the extracellular domain of the EGF receptor in various indications (e.g. EGFR vIII displaying exon 2-7 deletions). Other mutations such as the T790M point mutation in exon 20 as well as certain exon 20 insertions (e.g. D770_N771insNPG) which confer resistance to particular drugs should also be included, as well as double mutants such as the combined L858R / T790M mutation or the exon-19-del/T790M.
The expression "patient" should be understood to include also such cancer patients carrying tumors with wild-type HER2 receptor as well as pre-selected cancer patients with tumors harboring activating HER2 mutations, e.g. M774_A775insAYVM.

The indication "cancer" as used in the context of the invention is to be understood in a most general sense as a disease characterized by inappropriate cellular proliferation, migration, apoptosis or angiogenesis, preferably by inappropriate cellular proliferation. Inappropriate cell proliferation means cellular proliferation resulting from inappropriate cell growth, from excessive cell division, from cell division at an accelerated rate and/or from inappropriate cell survival.

"Radiotherapy" means administering ionizing radiation to the patient, as conventionally used in cancer therapy. Radiotherapy may be applied before, in parallel or after co-treatment by administration of the actives **1** and **2**.

"Tumour resection by surgery" is one standard option in cancer therapy and may be applied before or after co-treatment by administration of the actives **1** and **2**.

A first aspect of the present invention therefore is a pharmaceutical composition for the treatment of cancer comprising effective amounts of:
(1) a compound **1**:
   4-[(3-chloro-4-fluorophenyl)amino]-6-{[4-(N,N-dimethylamino)-1-oxo-2-buten-1-yl]amino}-7-((*S*)-tetrahydrofuran-3-yloxy)-quinazoline dimaleate; and
(2) at least a further chemotherapeutic agent **2** selected from the group consisting of docetaxel and paclitaxel;
optionally in combination with one or more pharmaceutically acceptable excipients, and optionally adapted for a co-treatment with radiotherapy or radio-immunotherapy, in the form of a combined preparation for simultaneous, separate or sequential use in the treatment of diseases involving cell proliferation, migration or apoptosis of cancer cells, or angiogenesis, preferably involving cell proliferation or apoptosis of cancer cells.

A second aspect of the present invention is directed to the use of compound **1**:
4-[(3-chloro-4-fluorophenyl)amino]-6-{[4-(N,N-dimethylamino)-1-oxo-2-buten-1-yl]-amino}-7-((*S*)-tetrahydrofuran-3-yloxy)-quinazoline dimaleate
for the manufacture of a pharmaceutical composition for the treatment of cancer, preferably for the treatment of the specific cancer-subindications referred to hereinafter, comprising effective amounts of:
   (1) compound **1**:
      4-[(3-chloro-4-fluorophenyl)amino]-6-{[4-(N,N-dimethylamino)-1-oxo-2-buten-1-yl]amino}-7-((*S*)-tetrahydrofuran-3-yloxy)-quinazoline dimaleate; and
   (2) at least a further chemotherapeutic agent **2** selected from the group consisting of docetaxel and paclitaxel;
optionally in combination with one or more pharmaceutically acceptable excipients, and optionally adapted for a co-treatment with radiotherapy or radio-immunotherapy, in the form of a combined preparation for simultaneous, separate or sequential use in the treatment of diseases involving cell proliferation, migration or apoptosis of cancer cells, or angiogenesis, preferably involving cell proliferation or apoptosis of cancer cells.

The expression "a pharmaceutical composition for the treatment of cancer" should be understood interchangeable with "a medicament for the treatment of cancer".

### Detailed Description of the Invention

In the context of the instant invention the indication "cancer" preferably is selected from the group consisting of solid tumours, e.g. from the group consisting of carcinomas, sarcomas, melanomas, myelomas, hematological neoplasias, lymphomas and childhood cancers.

Examples of carcinomas in the context of the invention include but are not limited to the group consisting of adenocarcinoma (AC), squamous cell carcinoma (SCC) and mixed or undifferentiated carcinomas. Carcinomas within the scope of the invention include but are not limited to the following histologies:
- Head and neck tumours: SCC, AC, transitional cell cancers, mucoepidermoid cancers, undifferentiated carcinomas;
- Central nervous system tumours: Astrocytoma, glioblastoma, meningeoma, neurinoma, schwannoma, ependymoma, hypophysoma, oligodendroglioma, medulloblastoma;
- Bronchial and mediastinal tumours:
   ∘ Bronchial tumours:
      ▪ Small cell lung cancers (SCLC): oat-cell lung cancer, intermediate cell cancer, combined oat-cell lung cancer;
      ▪ Non-small cell lung cancers (NSCLC): SCC, spindle cell carcinoma, AC, bronchioalveolar carcinoma, large cell NSCLC, clear cell NSCLC;
   ∘ Mesothelioma;
   ∘ Thymoma;
   ∘ Thyroid carcinomas: papillary, follicular, anaplastic, medullary;
- Tumours of the gastrointestinal tract:
   ∘ Oesophageal cancers: SCC, AC, anaplastic, carcinoid, sarcoma;
   ∘ Gastric cancers: AC, adenosquamous, anaplastic;
   ∘ Colorectal cancers: AC, including hereditary forms of AC, carcinoid, sarcoma;
   ∘ Anal cancers: SCC, transitional epithelial cancer, AC, basal cell carcinoma;
   ∘ Pancreatic cancers: AC, including ductal and acinary cancers, papillary, adenosquamous, undifferentiated, tumours of the endocrine pancreas;
   ∘ Hepatocellular carcinoma, cholangiocarcinoma, angiosarcoma, hepatoblastoma;
   ∘ Biliary carcinomas: AC, SCC, small cell, undifferentiated;
   ∘ Gastrointestinal stroma tumours (GIST);
- Gynaecological cancers:
   ∘ Breast cancers: AC, including invasive ductal, lobular and medullary cancers, tubular, mucinous cancers, Paget-carcinoma, inflammatory carcinoma, ductal and lobular carcinoma in situ;
   ∘ Ovarian cancers: Epithelial tumours, stroma tumours, germ cell tumours, undifferentiated tumours;
   ∘ Cervical cancers: SCC, AC, mixed and undifferentiated tumours;
   ∘ Endometrial cancers: AC, SCC, mixed, undifferentiated tumours;
   ∘ Vulvar cancers: SCC, AC;
   ∘ Vaginal cancers: SCC, AC;
- Urinary tract and testicular cancers:
   ∘ Testicular cancers: seminoma;
   ∘ Non-seminomatous germ cell tumours: teratoma, embryonal cell carcinoma, choriocarcinoma, yolk sac tumour, mixed, Sertoli and Leydig-cell tumours;
   ∘ Extragonadal germ cell tumours;
   ∘ Prostate cancers: AC, small cell, SCC;
   ∘ Renal cell cancers: AC, including clear cell, papillary and chromophobous carcinomas, hereditary forms (e.g. von-Hippel-Lindau syndrome), nephroblastoma;
   ∘ Urinary bladder cancers: transitional cell (urothelial) cancers, SCC, AC;
   ∘ Urethral cancers: SCC, transitional cell cancers, AC;
   ∘ Penile cancers: SCC;
- Tumours of endocrine tissue:
   ∘ Thyroid cancers: papillary, follicular, anaplastic, medullary carcinomas, including MEN syndrome;
   ∘ Tumours of the endocrine pancreas;
   ∘ Carcinoids;
   ∘ Pheochromocytoma.

Examples of sarcomas include Ewing-sarcoma, osteosarcoma or osteogenic sarcoma, chondrosarcoma, synovial sarcoma, leiomyosarcoma, rhabdomyosarcoma, mesothelial sarcoma or mesothelioma, fibrosarcoma, angiosarcoma or hemangioendothelioma, liposarcoma, glioma or astrocytoma, myxosarcoma, malignant fibrous histiocytoma, mesenchymous or mixed mesodermal tumour, neuroblastoma and clear cell sarcoma.

Examples of melanomas include to superficial spreading melanoma, nodular and lentigo-maligna melanoma.

Examples of myelomas include immunocytoma, plasmocytoma and multiple myeloma.

In one preferred embodiment the invention relates to the use according to the invention, wherein the hematological neoplasia is leukemia.

Examples of hematologic neoplasias include acute or chronic leukemias of myeloid, erythroid or lymphatic origin, myelodysplastic syndromes (MDS) and myeloproliferative syndromes (MPS, such as chronic myelogeneous leukemia, osteomyelofibrosis, polycythemia vera or essential thrombocythemia).

Examples of lymphomas include:
- Hodgkin's-lymphoma;
- Non-Hodgkin's-lymphomas: T- and B-cell lymphomas
   ∘ B-celllymphomas:
      ▪ Low and intermediate grade: Chronic lymphocytic leukemia (CLL), prolymphocytic leukemia (PLL), small lymphocytic lymphoma, hairy cell leukemia, plasmacytoid lymphoma, mantle cell lymphoma, follicular lymphoma, marginal zone lymphoma including MALT-lymphoma;
      ▪ High grade: diffuse large B-cell lymphoma (DLBCL including immunoblastic and centroblastic variants), lymphoblastic, Burkitt's lymphoma;
   ∘ T-cell lymphomas:
      ▪ Low grade: T-CLL, T-PLL, Mycosis fungoides, Sezary-syndrome;
      ▪ High grade: Anaplastic large cell, T-immunoblastic and lymphoblastic.

In another preferred embodiment the invention relates to the use according to the invention, wherein the disease is cancer selected from the group consisting of mixed tumours, undifferentiated tumours and metastases thereof.

Examples of mixed tumours include adenosquamous carcinomas, mixed mesodermal tumours, carcinosarcomas and teratocarcinomas.

Examples of undifferentiated, other tumours or metastases thereof include undifferentiated tumours, carcinomas of unknown primary (CUP), metastases of unknown primary (MUP) and pheochromocytoma, carcinoids.

Additionally the following tumour diseases which can be treated with a pharmaceutical composition in accordance with the invention are summarized:
acral lentiginous melanoma, actinic keratoses, adenoid cycstic carcinoma, adenomas, adenosarcoma, adrenocortical carcinoma, AIDS-related lymphoma, bartholin gland carcinoma, brain stem glioma, capillary carcinoma, central nervous system lymphoma, chondosarcoma, choriod plexus papilloma/carcinoma, cystadenoma, endodermal sinus tumor, endometrial hyperplasia, endometrial stromal sarcoma, endometrioid adenocarcinoma, epitheloid, focal nodular hyperplasia, gastrinoma, gestational trophoblastic tumor, glucagonoma, hepatic adenoma, hepatic adenomatosis, hypopharyngeal cancer, hypothalamic and visual pathway glioma, insulinoma, intraepithelial neoplasia, interepithelial squamous cell neoplasia, intraocular invasive squamous cell carcinoma, large cell carcinoma, islet cell carcinoma, Kaposi's sarcoma, laryngeal cancer, leukemia-related disorders, lip and oral cavity cancer, malignant mesothelial tumors, malignant thymoma, medulloepithelioma, merkel cell carcinoma, mucoepidermoid carcinoma, multiple myeloma/plasma cell neoplasm, mycosis fungoides, myelodysplastic syndrome, myeloproliferative disorders, nasal cavity and paranasal sinus cancer, nasopharyngeal cancer, neuroepithelial adenocarcinoma, nodular melanoma, oat cell carcinoma, oligodendroglial, oral cancer, oropharyngeal cancer, pineal cell, pituitary tumors, pseudosarcoma, pulmonary blastoma, parathyroid cancer, pineal and supratentorial primitive neuroectodermal tumors, pituitary tumor, plasma cell neoplasm, pleuropulmonary blastoma, retinoblastoma, serous carcinoma, small intestine cancer, soft tissue carcinomas, somatostatin-secreting tumor, supratentorial primitive neuroectodermal tumors, uveal melanoma, verrucous carcinoma, vipoma, Waldenstrom's macroglobulinemia, well differentiated carcinoma, and Wilm's tumor.

It is known that cancer patients carrying activating EGFR mutations in their tumors, i. e. within the tyrosine kinase domain of the EGF receptor, may show increased sensitivity to treatment with EGFR inhibitors. Analogously, cancer patients carrying activating HER2 mutations, e.g. M774_A775insAYVM, in their tumors may show increased sensitivity to treatment with HER2 inhibitors. Both groups of patients as well as a subgroup carrying both activating EGFR and HER2 mutations may show increased sensitivity to treatment with dual inhibitors of erbbl receptor (EGFR) and erbB2 (Her2/neu).

The presence of specific gain-of-function mutations within the tyrosine kinase domain of the EGF receptor in a subgroup of NSCLC patients has been associated with increased sensitivity to treatment with gefitinib and erlotinib (Lynch, New England Journal Medicine 350, 2129 (2004); Paez, Science 304, 1497 (2004); Pao, Proceedings of the National Academy of Science of the United States 101, 13306 (2004)). In particular, the L858R point mutation (exon 21) as well as deletion/insertion mutations in the ELREA sequence (exon 19) account for the majority of gefitinib responders. A secondary point mutation in exon 20, T790M, is associated with acquired resistance to gefitinib or erlotinib. This mutation is analogous to the T315I mutation identified in CML patients who relapse under imatinib treatment (imatinib resistant patients).

Irreversible inhibitors (e.g., HKI-272 or CL 387,785), in contrast to reversible inhibitors (e.g., gefitinib), are able to inhibit proliferation and EGF-induced EGFR phosphorylation in cell lines expressing double mutant EGF receptors (Kwak, Proceedings of the National Academy of Science of the United States 102, 7665 (2005) and Kobayashi, New England Journal Medicine 352, 786 (2005)).

The context of the present invention therefore includes, as a sub-aspect, optional pre-selection of cancer patients for an EGFR mutation in the tyrosine kinase domain of the EGF receptor as well as pre-selection of cancer patients for an HER2 mutation. The EGFR mutations preferably relevant in in this context are selected from the group consisting of the L858R and L861 point mutations in the activation loop (exon 21), in-frame deletion/insertion mutations in the ELREA sequence (exon 19), substitutions in G719 situated in the nucleotide binding loop (exon 18), activating mutations in the extracellular domain of the EGF receptor such as EGFR vIII displaying exon 2-7 deletions, the T790M point mutation in exon 20, exon 20 insertions such as D770_N771insNPG, and double mutants such as the combined L858R / T790M mutation and the exon-19-del/T790M. The HER2 mutation preferably relevant in in this context is the M774_A775insAYVM mutation.

Methods for detecting mutations in the tyrosine kinase domain of the EGF receptor are kown in the art, several corresponding diagnostic tools are approved by the FDA and commercially available, e.g. an assay for the detection of epidermal growth factor receptor mutations in patients with non-small cell lung cancer (Genzyme Corp.; see also Journal of Clinical Oncology, 2006 ASCO Annual Meeting Proceedings (Post-Meeting Edition). Vol 24, No 18S (June 20 Supplement), 2006: Abstract 10060).

The second aspect of the invention, directed to the use of compound **1** for the manufacture of a pharmaceutical composition comprising effective amounts of compound **1** and of at least a further chemotherapeutic agent **2** selected from docetaxel and paclitaxel for the treatment of cancer, applies accordingly to the optional sub-aspect of pre-selection of cancer patients for an activating EGFR mutation in the tyrosine kinase domain of the EGF receptor and/or pre-selection of cancer patients for an activating HER2 mutation.

### Method of treatment:

The term "therapeutically effective amount" shall mean that amount of a drug or pharmaceutical agent that will elicit the biological or medical response of a tissue, system, animal or human that is being sought by a researcher or clinician.

The elements of the combination of **1** and **2** may be administered by oral (including buccal or sublingual), enterical, parenteral (e.g., intramuscular, intraperitoneal, intravenous, transdermal or subcutaneous injection, or implant), nasal, vaginal, rectal, or topical (e.g. inhalative) routes of administration and may be formulated, alone or together, in suitable dosage unit formulations containing conventional non-toxic pharmaceutically acceptable carriers, adjuvants and vehicles appropriate for each route of administration.

In a preferred embodiment the element **1** of the combination in accordance with the invention is administered orally, enterically, transdermally, intravenously, peritoneally or by injection, preferably orally.

### Dosages / compound 1:

Compound **1**, or its polymorph, hydrate or solvate, is administered intermittent or in a daily dosage such that the plasma level of the active substance preferably lies between 10 and 5000 nM for at least 12 hours of the dosing interval.

Compound **1** may be administered to the human patient in a daily dose of 0.01-4 mg/kg of body weight (bw), preferably 0.1-2 mg/kg, particularly preferred in a dose of 0.2-1.3 mg/kg bw. For oral treatment compound **1** may be administered daily in a total dose of 10, 20, 30, 40, 50, 60, 70, 100, 200, or 300 mg, optionally divided into multiple doses, e.g. 1 to 3 doses to be administered through the day, preferably administered only once a time, and applied as an equivalent dose containing respective amounts of the active base component. Especially for higher doses periods of treatment should alternate with periods of recovery, without administering compound **1**. For instance, treatment could follow a "7 day on - 7 day off", a "14 day on - 14 day off", a "21 day on 7 day off' or a continuous dosing schedule. "On-off' time periods can be chosen shorter, especially if higher doses are administered, or individually adapted to the needs of the patient.

However, it may optionally be necessary to deviate from the amounts specified, depending on the body weight or method of administration, the individual response to the medication, the nature of the formulation used and the time or interval over which it is administered. Thus, in some cases, it may be sufficient to use less than the minimum quantity specified above, while in other cases the upper limit specified will have to be exceeded. When large amounts are administered it may be advisable to spread them over the day in a number of single doses.

### Dosages / chemotherapeutic agents 2:

Dosages and treatment schedules for the individual chemotherapeutic agents **2** are known in the art and may be applied analogously within the invention. Depending on the individual activity of the specific combination dosage of the chemotherapeutic agents **2** may be reduced, e.g. may vary in the range of 1/1 to 1/20 of the dosages described in the prior art.

For patients with metastatic breast cancer the combination with docetaxel may be given at a dose between 55 mg/m² and 100 mg/m² and most specifically at a dose of 60 to 75 mg/m² in administration schedule of once every 21 days. In a weekly administration schedule the dose of docetaxel may be lowered.

A similar dose range of docetaxel will be used in the treatment of hormone-refractory prostate cancer. In this case docetaxel is administered together with daily prednisone and/or with the administration of estramustine. The dose of estramustine is 14 mg per kg of body weight given in 3 or 4 divided doses daily. Most patients are treated at a dose range between 10 and 16 mg/kg body weight.

Docetaxel is also used in the treatment of non-small cell lung cancer at similar doses and schedules.

In patients with metastatic breast cancer, the administration of paclitaxel is at a dose of up to 175 mg/m² over 3 hours every 3 weeks. In a weekly administration schedule paclitaxel dose may be lower. In an adjuvant setting, paclitaxel will be administered at doses up to 175 mg/m² over 3 hours every 3 weeks sequentially to a combination with a doxorubicin-containing chemotherapy (four courses of doxorubicin and cyclophosphamide were used).

For patients with non-small cell lung cancer the recommended dose of paclitaxel is 135 mg/m² IV over 24 hours every 3 weeks. The administration of paclitaxel is followed by cisplatin at 75 mg/m². Another option is the combination of paclitaxel with carboplatin.

In patients with ovarian carcinoma, paclitaxel is used at a dose of 175 mg/m² IV over 3 hours followed by cisplatin at 75 mg/m² or at a dose of 135 mg/m² over 24 hours followed by cisplatin at a dose of 75 mg/m². Paclitaxel can also be combined with carboplatin. This cycle will be repeated every 3 weeks. Another treatment schedule in the more advanced disease setting is the administration of paclitaxel at either 135 or 175 mg/m² IV over 3 hours every 3 weeks.

However, it may optionally be necessary to deviate from the amounts specified, depending on the body weight or method of administration, the individual response to the medication, the nature of the formulation used and the time or interval over which it is administered. Thus, in some cases, it may be sufficient to use less than the minimum quantity specified above, while in other cases the upper limit specified will have to be exceeded. When large amounts are administered it may be advisable to spread them over the day in a number of single doses.

### Dosages / radiotherapy or radio-immunotherapy:

Dosages and treatment schedules for radiotherapy and radio-immunotherapy are known in the art and may be applied analogously in the context of the invention. Depending on the individual activity of the specific combination with compound **1** and, optionally, chemotherapeutic agent **2**, dosage of the radiotherapy and radio-immunotherapy component may be reduced, e.g. may vary in the range of 1/1 to 1/20 of the dosages described in the prior art.

### Pharmaceutical compositions:

As used herein, the term "composition" is intended to encompass a product comprising the specified ingredients in the specified amounts, as well as any product which results, directly or indirectly, from a combination of the specified ingredients in the specified amounts. The amount of pharmaceutically active compound in each case should be in the range from 0.1 - 90 wt.%, preferably 0.5 - 50 wt.% of the total composition, i.e. in amounts which are sufficient to achieve the dosage ranges given hereinbefore. The doses specified may, if necessary, be given several times a day.

As already mentioned before, within the context of the present invention, the components **1** and **2** of the composition for a combination therapy may be administered separately (which implies that they are formulated separately) or together (which implies that they are formulated together). Hence, the administration of one element of the combination of the present invention may be prior to, concurrent to, or subsequent to the administration of the other element of the combination.

One embodiment of the invention relates to a pharmaceutical combination preparation kit for the treatment of cancer diseases, comprising
(i) a first compartment containing a pharmaceutical composition comprising a therapeutically effective amount of a compound **1**:
   4-[(3-chloro-4-fluorophenyl)amino]-6-{[4-(N,N-dimethylamino)-1-oxo-2-buten-1-yl]amino}-7-((*S*)-tetrahydrofuran-3-yloxy)-quinazoline dimaleate; and
(ii) a second containment containing a pharmaceutical composition comprising a therapeutically effective amount of at least a further chemotherapeutic agent **2** selected from the group consisting of docetaxel and paclitaxel,
said kit being optionally adapted for a co-treatment with radiotherapy or radio-immunotherapy.

In a preferred embodiment the invention relates to a pharmaceutical combination preparation kit, wherein the formulation of the compound **1** in accordance with the present invention is for oral administration.

The pharmaceutical compositions for the administration of the components 1 and **2** of this invention may conveniently be presented in dosage unit form and may be prepared by any of the methods well known in the art of pharmacy. All methods include the step of bringing the active ingredient into association with the carrier which is constituted of one or more accessory ingredients. In general, the pharmaceutical compositions are prepared by uniformly and intimately bringing the active ingredients into association with a liquid carrier or a finely divided solid carrier or both, and then, if necessary, shaping the product into the desired dosage form. In the pharmaceutical compositions the active compounds are included in an amount sufficient to produce the desired pharmacologic effect.

Dosage forms intended for oral use may be prepared according to any method known to the art for the manufacture of pharmaceutical formulations and such compositions. The excipients used may be, for example: (a) inert diluents such as mannitol, sorbitol, calcium carbonate, pregelatinized starch, lactose, calcium phosphate or sodium phosphate; (b) granulating and disintegrating agents, such as povidone, copovidone, hydroxypropylmethylcellulose, corn starch, alginic acid, crospovidone, sodiumstarchglycolate, croscarmellose, or polacrilin potassium; (c) binding agents such as microcrystalline cellulose or acacia; and (d) lubricating agents such as magnesium stearate, stearic acid, fumaric acid or talc.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number of layers to achieve delayed release, possibly using the excipients mentioned above for the tablets.

The dosage of the active ingredients in the compositions in accordance with the present invention may be varied, although the amount of the active ingredients **1** and **2** shall be such that a suitable dosage form is obtained. Hence, the selected dosage and the selected dosage form shall depend on the desired therapeutic effect, the route of administration and the duration of the treatment. Suitable dosage ranges for the combination are from the maximal tolerated dose for the single agent to lower doses, e.g. to one tenth of the maximal tolerated dose.

The following Examples serve to illustrate the invention without restricting it:

### Example 1: Ovarian Cancer SKOV-3 Xenografts in Mice

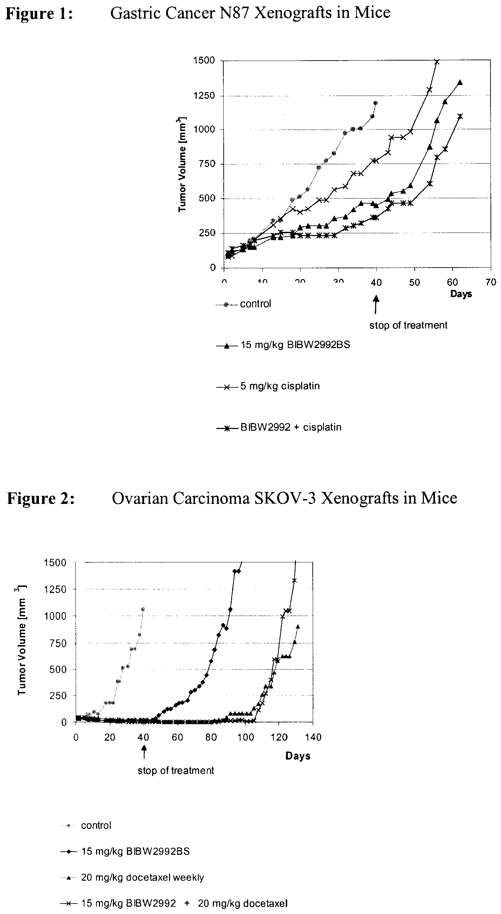

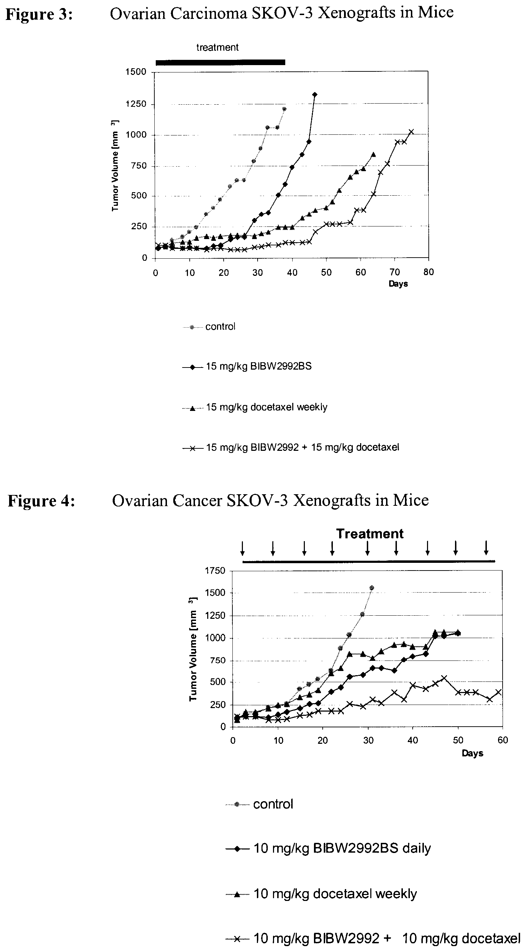

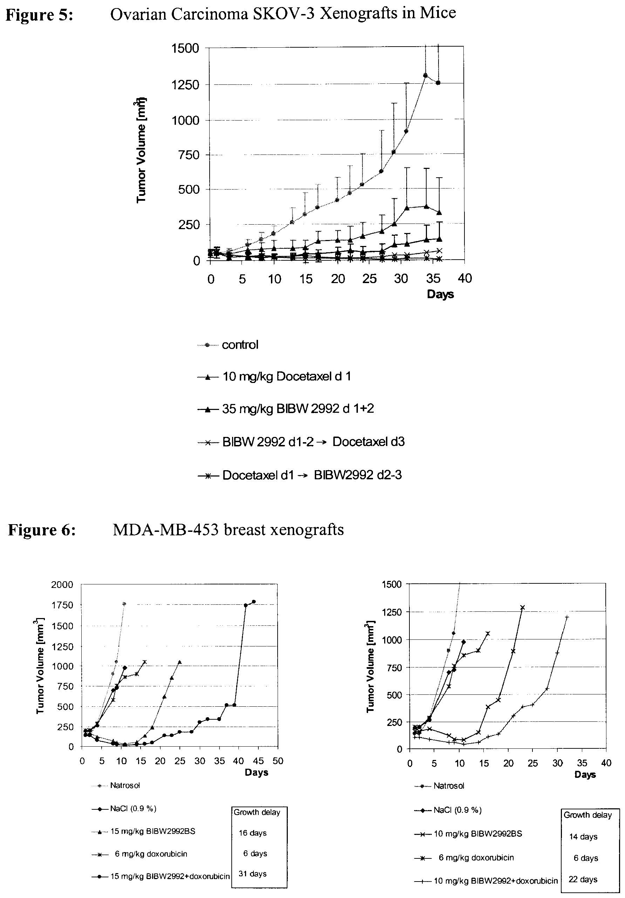

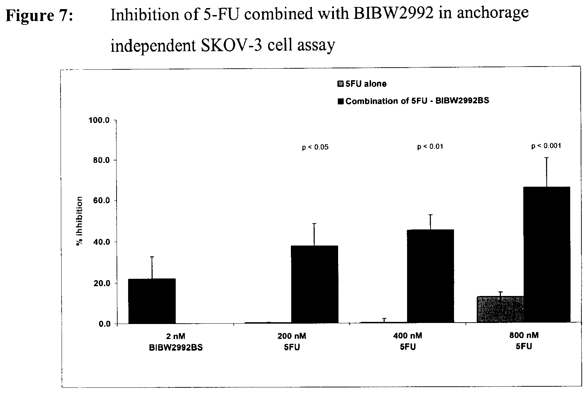

Results of an experiment comparing daily treatment of ovarian cancer SKOV-3 xenografts in mice with BIBW2992 alone (10 mg/kg), once weekly treatment with docetaxel alone (10 mg/kg) and the combination of BIBW2992 / docetaxel (10 mg/kg / (10 mg/kg) are shown in **Figure 1** (Appendix). Animals were treted until the end of the experiment. Combination treatment significantly delayed tumor growth compared to single agent treatment. Therefore a clear advantage of combination treatment could be shown compared to single agent treatment with BIBW 2992 or docetaxel.

### Example 2: Ovarian Carcinoma SKOV-3 Xenografts in Mice

Results of an experiment comparing treatment of ovarian cancer SKOV-3 xenografts in mice with BIBW2992 alone (35 mg/kg), administered twice weekly on two consecutive days, docetaxel alone (10 mg/kg), given once weekly and the pulsatile combination of BIBW2992 and docetaxel are shown in **Figure 2** (Appendix). For the combinations BIBW 2992 was administered for two consecutive days (day 1-2) followed by a single administration of docetaxel on day 3 or docetaxel was given on day 1 followed by BIBW 2992 on two consecutive days (day 2-3). Treatment cycles were repeated weekly throughout the experiment. This study clearly shows that the combination treatments with BIBW 2992 and docetaxel resulted in better anti-tumor effects than either drug alone. Furthermore, the data demonstrate that docetaxel administration followed by BIBW 2992 on two consecutive days results in better and persistent anti-tumor activity than the inverse schedule.

The following Examples 3 to 7 contain as active substance compound **1**.

### Example 3: Coated immediate-release tablets containing 75 mg of active substance by dry-granulation process

### Composition:

**1 tablet contains:**

| | |
|---|---|
| active substance | 75.0 mg |
| calcium phosphate anhydrous | 108.0 mg |
| corn starch | 35.5 mg |
| polyvinylpyrrolidone | 10.0 mg |
| magnesium stearate | 1.5 mg |
| hydroxypropylmethylcellulose | 7.5 mg |
| polyethylene glycol | 1.0 mg |
| polydextrose | 5.0 mg |
| talc | 1.0 mg |
| pigments | 0.5 mg |
| water (volatile) | *** |
| | 245.0 mg |

### Preparation:

The active substance is mixed with calcium phosphate, corn starch, polyvinylpyrrolidone, hydroxypropylmethylcellulose and half the specified amount of magnesium stearate. Ribbons are produced in a roller-compactor and these are then rubbed through a screen with a mesh size of 1.5 mm using a suitable machine and mixed with the rest of the magnesium stearate. This granulate is compressed in a tablet-making machine to form tablets of the desired shape.
Weight of core: 230 mg
Tablet shape: 9 mm round, bi- convex

The tablet cores are subsequently coated with an aqueous film-coat consisting essentially of hydroxypropylmethylcellulose, polyethylene glycol, polydextrose, talc and pigments.
Weight of coated tablet: 245 mg.

### Example 4: Extended- release tablets containing 100 mg of active substance by organic granulation granulation process

**1 tablet contains:**

| | |
|---|---|
| active substance | 100.0 mg |
| lactose | 34.0 mg |
| hydroxypropylmethylcellulose | 80 mg |
| polyvinylpyrrolidone | 4.0 mg |
| magnesium stearate | 2.0 mg |
| ethanol (volatile) | *** |
| | 220.0 mg |

### Preparation:

The active substance, lactose and hydroxypropylmethylcellulose are mixed together and uniformly moistened with solution of the polyvinylpyrrolidone in ethanol. After the moist composition has been screened (2.0 mm mesh size) and dried in a rack-type drier at 50°C it is screened again (1.5 mm mesh size) and the lubricant is added. The final blend is compressed to form tablets.

| | |
|---|---|
| Weight of tablet: | 220 mg |
| Tablet shape: | 10 mm, flat-faced, with bevelled edges. |

### Example 5: Tablets containing 150 mg of active substance by aqueous granulation process

**1 tablet contains:**

| | |
|---|---|
| active substance | 150.0 mg |
| powdered lactose | 98.0mg |
| corn starch | 40.0 mg |
| colloidal silica | 1.0 mg |
| polyvinylpyrrolidone | 10.0 mg |
| magnesium stearate | 1.0 mg |
| | 300.0 mg |

### Preparation:

The active substance mixed with lactose, corn starch is moistened with a 20% aqueous polyvinylpyrrolidone solution and passed through a screen with a mesh size of 1.5 mm. The granules, dried at 45°C, are passed through the same screen again and mixed with the specified amount of magnesium stearate and colloidal silica. Tablets are pressed from thefinal blend.

| | |
|---|---|
| Weight of tablet: | 300 mg |
| Tablet shape: | 14 mm x 6.8 mm, oblong biconvex with embossement |

### Example 6: Hard capsules containing 150 mg of active substance in granules

### Composition:

**1 capsule contains:**

| | |
|---|---|
| active substance | 150.0 mg |
| microcrystalline cellulose | 80.0 mg |
| lactose (spray-dried) | 87.0 mg |
| colloidal silica | 10.0 mg |
| | 320.0 mg |

### Preparation:

The active substance is mixed with the excipients in a high-shear mixer, passed through a screen with a mesh size of 0.75 mm and homogeneously mixed using a suitable apparatus. The finished mixture is packed into size 1 hard gelatin capsules.

| | |
|---|---|
| Capsule filling: | 320 mg |
| Capsule shape: | size 1, opaque hard capsule. |

### Example 7:

### Suppositories containing 150 mg of active substance

### Composition:

**1 suppository contains:**

| | |
|---|---|
| active substance | 150.0 mg |
| polyethyleneglycol 1500 | 550.0 mg |
| polyethyleneglycol 6000 | 460.0 mg |
| polyoxyethylene sorbitan | |
| monostearate | 840.0 mg |
| | 2,000.0 mg |

### Preparation:

After the suppository mass has been melted the active substance is homogeneously suspended therein and the melt is poured into chilled moulds.

## Claims

1. A pharmaceutical composition comprising effective amounts of:
(1) a compound **1:**
4-[(3-chloro-4-fluorophenyl)amino]-6-{[4-(N,N-dimethylamino)-1-oxo-2-buten-1-yl]amino}-7-((*S*)-tetrahydrofuran-3-yloxy)-quinazoline dimaleate; and
(2) at least a further chemotherapeutic agent **2** selected from the group consisting of docetaxel and paclitaxel;
optionally in combination with one or more pharmaceutically acceptable excipients,
and optionally adapted for a co-treatment with radiotherapy or radio-immunotherapy, in the form of a combined preparation for simultaneous, separate or sequential use.

2. A pharmaceutical composition according to claim 1, in the form of a combined preparation kit for the treatment of cancer diseases, comprising
(i) a first compartment containing a pharmaceutical composition comprising a therapeutically effective amount of a compound **1:**
4-[(3-chloro-4-fluorophenyl)amino]-6-{[4-(N,N-dimethylamino)-1-oxo-2-buten-1-yl]amino}-7-((*S*)-tetrahydrofuran-3-yloxy)-quinazoline dimaleate;
and
(ii) a second containment containing a pharmaceutical composition comprising a therapeutically effective amount of at least a further chemotherapeutic agent **2** as defined in claim 1;
said kit being optionally adapted for a co-treatment with radiotherapy or radio-immunotherapy.

3. Use of a compound **1**:
4-[(3-chloro-4-fluorophenyl)amino]-6-{[4-(N,N-dimethylamino)-1-oxo-2-buten-1-yl]-amino}-7-((*S*)-tetrahydrofuran-3-yloxy)-quinazoline dimaleate;
for the manufacture of a pharmaceutical composition for the treatment of a patient suffering from cancer, comprising effective amounts of:
(1) a compound **1**:
4-[(3-chloro-4-fluorophenyl)amino]-6-{[4-(N,N-dimethylamino)-1-oxo-2-buten-1-yl]-amino}-7-((*S*)-tetrahydrofuran-3-yloxy)-quinazoline dimaleate; and
(2) at least a further chemotherapeutic agent **2** as defined in claim 1;
optionally in combination with one or more pharmaceutically acceptable excipients, and optionally adapted for a co-treatment with radiotherapy or radio-immunotherapy,
in the form of a combined preparation for simultaneous, separate or sequential use in the treatment of a patient suffering from cancer.

4. The use of claim 3, wherein the patient is a pre-selected cancer patient shown to carry a tumor harboring an activating EGFR mutation.

5. The use of claim 3, wherein the EGFR mutation is selected from the group consisting of the L858R point mutation, deletion/insertion mutations in the ELREA sequence, the T790M point mutation in exon 20, and double mutations such as the combined L858R / T790M mutation.

6. The use of claim 3, wherein the patient is a pre-selected cancer patient shown to carry a tumor harboring an activating HER2 mutation.

7. The use of claim 6, wherein the HER2 mutation is the M774_A775insAYVM mutation.

## Patentansprüche

1. Pharmazeutische Zusammensetzung umfassend wirksame Mengen von:
(1) einer Verbindung **1**:
4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin-Dimaleat; und
(2) mindestens einem weiteren chemotherapeutischen Mittel **2,** ausgewählt aus der Gruppe bestehend aus Docetaxel und Paclitaxel;
gegebenenfalls in Kombination mit einem oder mehreren pharmazeutisch akzeptablen bzw. annehmbaren Hilfsstoffen,
und gegebenenfalls angepasst an eine Co-Behandlung mit Radiotherapie oder Radioimmuntherapie, in Form einer kombinierten Zubereitung für die gleichzeitige, getrennte oder aufeinander folgende Verwendung.

2. Pharmazeutische Zusammensetzung gemäß Anspruch 1, in Form eines kombinierten Zubereitungskits zur Behandlung von Krebserkrankungen, umfassend
(i) ein erstes Behältnis, enthaltend eine pharmazeutische Zusammensetzung umfassend eine therapeutisch wirksame Menge einer Verbindung **1:**
4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin-Dimaleat;
und
(ii) ein zweites Behältnis, enthaltend eine pharmazeutische Zusammensetzung umfassend eine therapeutisch wirksame Menge von mindestens einem weiteren chemotherapeutischen Mittel **2**, das wie in Anspruch 1 definiert ist;
wobei besagter Kit gegebenenfalls an die Co-Behandlung mit Radiotherapie oder Radioimmuntherapie angepasst ist.

3. Verwendung einer Verbindung **1**:
4-[(3-Chlor-4-fluorphenyl)amino]-6-{[4-(N,N-dimethylamino)-1-oxo-2-buten-1-yl]-amino}-7-((*S*)-tetrahydrofuran-3-yloxy)-chinazolin-Dimaleat;
zur Herstellung eines Arzneimittels zur Behandlung eines an Krebs leidenden Patienten, umfassend wirksame Mengen von:
(1) einer Verbindung **1**:
4-[(3-Chlor-4-fluorphenyl)amino]-6-{[4-(N,N-dimethylamino)-1-oxo-2-buten-1-yl]-amino}-7-((*S*)-tetrahydrofuran-3-yloxy)-chinazolin-Dimaleat; und
(2) mindestens einem weiteren chemotherapeutischen Mittel **2**, das wie in Anspruch 1 definiert ist;
gegebenenfalls in Kombination mit einem oder mehreren pharmazeutisch akzeptablen bzw. annehmbaren Hilfsstoffen, und gegebenenfalls angepasst an eine Co-Behandlung mit Radiotherapie oder Radioimmuntherapie,
in Form einer kombinierten Zubereitung für die gleichzeitige, getrennte oder aufeinander folgende Verwendung in der Behandlung eines an Krebs leidenden Patienten.

4. Verwendung gemäß Anspruch 3, wobei der Patient ein vorausgewählter Krebspatient ist, bei dem gezeigt wurde, dass er einen Tumor hat, der eine aktivierende EGFR-Mutation aufweist.

5. Verwendung gemäß Anspruch 3, wobei die EGFR-Mutation ausgewählt ist aus der Gruppe, bestehend aus der L858R-Punktmutation, Deletions-/Insertions-Mutationen in der ELREA-Sequenz, der T790M-Punktmutation in Exon 20 und Doppelmutationen, wie der kombinierten L858R / T790M-Mutation.

6. Verwendung gemäß Anspruch 3, wobei der Patient ein vorausgewählter Krebspatient ist, bei dem gezeigt wurde, dass er einen Tumor hat, der eine aktivierende HER2-Mutation aufweist.

7. Verwendung gemäß Anspruch 6, wobei die HER2-Mutation die M774_A775insAYVM-Mutation ist.

## Revendications

1. Composition pharmaceutique comprenant des quantités efficaces:
(1) d'un composé **1**:
la dimaléate de 4-[(3-chloro-4-fluorophényl)amino]-6-{[4-(N,N-diméthylamino)-1-oxo-2-butén-1-yl]amino}-7-((*S*)-tétrahydrofuran-3-yloxy)-quinazoline; et
(2) d'au moins un agent chimiothérapeutique supplémentaire **2** sélectionné dans le groupe consistant en docétaxel et paclitaxel;
éventuellement en combinaison avec un ou plusieurs excipients pharmaceutiquement acceptables,
et éventuellement adaptée pour un cotraitement avec une radiothérapie ou une radio-immunothérapie, sous la forme d'une préparation combinée pour l'application simultanée, séparée ou séquentielle.

2. Composition pharmaceutique selon la revendication 1, sous la forme d'un kit de préparation combinée destinée au traitement de maladies cancéreuses, comprenant
(i) un premier compartiment contenant une composition pharmaceutique comprenant une quantité thérapeutiquement efficace d'un composé 1:
la dimaléate de 4-[(3-chloro-4-fluorophényl)amino]-6-{[4-(N,N-diméthylamino)-1-oxo-2-butén-1-yl]amino} -7-((*S*)-tétrahydrofuran-3-yloxy)-quinazoline;
et
(ii) un second confinement contenant une composition pharmaceutique comprenant une quantité thérapeutiquement efficace d' au moins un agent chimiothérapeutique supplémentaire **2** tel que défini dans la revendication 1;
ledit kit étant éventuellement adapté pour un cotraitement avec une radiothérapie ou une radio -immunothérapie.

3. Utilisation d'un composé **1**:
la dimaléte de 4-[(3-chloro-4-fluorophényl)amino]-6-{[4-(N,N-diméthylamino)-1-oxo-2-butén-1-yl]amino}-7-((*S*)-tétrahydrofuran-3-yloxy)-quinazoline;
pour la fabrication d'une composition pharmaceutique destinée au traitement d'un patient souffrant du cancer, comprenant des quantités efficaces:
(1) d'un composé **1**:
la dimaléate de 4-[(3-chloro-4-fluorophényl)amino]-6-{[4-(N,N-diméthylamino)-1-oxo-2-butén-1-yl]amino}-7-((*S*)-tétrahydrofuran-3-yloxy)-quinazoline; et
(2) d'au moins un agent chimiothérapeutique supplémentaire **2** tel que défini dans la revendication 1;
éventuellement en combinaison avec un ou plusieurs excipients pharmaceutiquement acceptables, et
éventuellement adaptée pour un cotraitement avec une radiothérapie ou une radio-immunothérapie,
sous la forme d'une préparation combinée pour l'application simultanée, séparée ou séquentielle dans le traitement d'un patient souffrant du cancer.

4. Utilisation selon la revendication 3, où le patient est un patient cancéreux présélectionné qui a été démonstré comme étant porteur d'une tumeur abritant une mutation activatrice de l' EGFR.

5. Utilisation selon la revendication 3, où la mutation de l' EGFR est choisie dans le groupe constitant en la mutation ponctuelle L858R, des mutations par délétion/insertion dans la séquence ELREA, la mutation ponctuelle T790M dans l' exon 20, et des mutations doubles comme la mutation combinée L858R / T790M.

6. Utilisation selon la revendication 3, où le patient est un patient cancéreux présélectionné qui a été démonstré comme étant porteur d'une tumeur abritant une mutation activatrice de HER2.

7. Utilisation selon la revendication 6, où la mutation de HER2 est la mutation M774_A775insAYVM.
